# EUROPEAN PATENT SPECIFICATION

(11) **EP 1 095 006 B1**
(45) Date of publication and mention of the grant of the patent: **24.12.2008**
(21) Application number: 98936423.7
(22) Date of filing: 14.07.1998
(51) Int. Cl.: C07C 51/50, C07C 57/04, C07C 69/54, C07C 67/62, C07C 69/15, C07C 231/22, C07C 233/09, C07C 253/32, C07C 255/08, C07B 63/04, C07D 211/94

(54) **DERIVATIVES OF 1-OXYL-4-HYDROXY-2,2,6,6-TETRAMETHYLPIPERIDINE AS POLYMERIZATION INHIBITORS FOR (METH) ACRYLATE MONOMERS**
1-OXYL-4-HYDROXY-2,2,6,6-TETRAMETHYLPIPERIDINDERIVATE ALS POLYMERISATIONSINHIBITOREN FÜR (METH)ACRYLATMONOMERE
DERIVES DE 1-OXYL-4-HYDROXY-2,2,6,6-TETRAMETHYLPIPERIDINE UTILISES COMME INHIBITEURS DE POLYMERISATION POUR DES MONOMERES (METH)ACRYLATE

(43) Date of publication of application: 02.05.2001
(60) Divisional application: 08168030.8
(73) Proprietor: Ciba Holding Inc., 4057 Basel (CH)
(72) Inventor: CUNKLE, Glen, Thomas, Stamford, CT 06903-3827 (US); GANDE, Matthew, Edward, Danbury, CT 06811 (US); SELTZER, Raymond, New City, NY 10956-2539 (US); THOMPSON, Thomas, Friend, Highland Mills, NY 10930 (US)
(86) International application number: PCT/EP1998/004382
(87) International publication number: WO 2000/003965

(56) References cited:
- EP-A- 0 697 386
- WO-A-98/02400
- WO-A-98/56746
- WO-A-99/05108
- DE-A- 19 510 184
- DE-A- 19 609 312
- US-A- 5 322 960
- CHEMICAL ABSTRACTS, vol. 104, no. 17, 28 April 1986 Columbus, Ohio, US; abstract no. 148699w, BARACU I ET AL: "Potential anticancer agents. XXVI. Spin labeled nitrosoureas" page 677; XP002097007 & J. PRAKT. CHEM., vol. 327, no. 4, 1985, pages 667-74,

## Description

The instant invention pertains to the use of selected derivatives of 1-oxyl-2,2,6,6-tetramethyl-4-hydroxypiperidine as inhibitors for preventing the premature polymerization of acrylic and methacrylic acids, their esters and amides, of vinyl acetate and of acrylonitrile in the presence of water.

Many of the industrially important ethylenically unsaturated monomers are highly susceptible to unwanted radical polymerization initiated either thermally or by adventitious impurities. Some examples of these monomers are acrylic and methacrylic acid, acrylate and methacrylate esters, acrylamide and methacrylamide, vinyl acetate and acrylonitrile. Premature polymerization may occur during manufacture, purification or storage of the monomer. Many of these monomers are purified by distillation. It is in this operation where premature polymerization is most likely to occur and to be the most troublesome. Methods to prevent or reduce the amount of such polymerization are thus highly desirable since the prevention or mitigation of such premature polymerization increases the yield of purified monomer and also insures against costly and potentially dangerous runaway polymerization in the plant.

Stable nitroxides are known in the art to be effective in preventing the premature radical polymerization of ethylenically unsaturated monomers. Some examples are seen in Japanese Hei 9-268138 which discloses the stabilization of styrene by 1-oxyl-2,2,6,6-tetramethyl-4-hydroxypiperidine and its lower alkyl ethers in the presence of nitrophenols. United States Patent Nos. 3,747,988 describes the use of 1-oxyl-2,2,6,6-tetramethyl-4-hydroxypiperidine as a polymerization inhibitor for acrylonitrile in the presence of water and oxygen. United States Patent No. 3,488,338 discloses that 1-oxyl-2,2,6,6-tetramethyl-4-hydroxypiperidine is an effective chain stopper in the aqueous polymerization of chloroprene. British Patent No. 1,127,127 describes the stabilization of neat acrylic acid by 1-oxyl-2,2,6,6-tetramethyl-4-hydroxypiperidine. Japanese Sho 60-36501 describes the stabilization of acrylate and methacrylate esters.

United States Patent Nos. 5,322,960 and 5.504.243 disclose the use of 1-oxyl-2,2,6,6-tetramethyl-4-hydroxypiperidine in preventing the polymerization of acrylic and methacrylic acids and their esters in the presence of water, but tout the great advantages of using said oxyl compound in combination with manganese acetate, or with hydroquinone and phenothiazine.

EP 178,168 teaches the use of 1-oxyl-2,2,6,6-tetramethyl-4-hydroxypiperidine or its reaction product with hexamethylene diisocyanate in stabilizing acrylic acid or methacrylic acid in the presence of water.

EP 791,573 discloses that the lower alkyl or aryl esters of 1-oxyl-2,2,6,6-tetramethyl-4-hydroxypiperidine are effective polymerization inhibitors alone or in combination with various coadditives for vinyl acetate in the presence of water.

Japanese Hei 5-320205 generically describes the use of 1-oxyl-2,2,6,6-tetramethyl-4-hydroxypiperidine, its lower alkyl ethers and lower alkanoic esters in preventing the polymerization of acrylic and methacrylic acids alone, but preferably in the presence of chelating agents for ferric salts, such as ethylenediaminetetraacetic acid. The 4-hydroxy, 4-methoxy and 4-acetoxy derivatives are specifically disclosed.

Japanese Hei 5-320217 teaches the stabilization of acrylic and methacrylic acids with 1-oxyl-2,2,6,6-tetramethyl-4-hydroxypiperidine, its lower alkyl ethers and lower alkanoic esters alone, but preferably in the presence of phenothiazine, an aromatic amine or phenol. The 4-hydroxy, 4-methoxy and 4-acetoxy derivatives are specifically disclosed.

German Application DE 195 10 184 A1 describes amide and formamide derivatives of 1-oxyl-2,2,6,6-tetramethyl-4-aminopiperidine as stabilizers for radically polymerizable monomers. German Application DE196 09 312 A1 describes ester derivatives of 1-oxyl-2,2,6,6-tetramethyl-4.-hydroxypiperidine as stabilizers for radically polymerizable monomers. Both documents do not differentiate between aqueous and non-aqueous systems.

United States Patent No. 5,545,786 describes the use of selected nitroxide compounds in the prevention of the premature polymerization of vinyl aromatic monomers such as styrene especially in the presence of oxygen. There is no disclosure or suggestion that such nitroxide compounds would be particularly effective in stabilizing acrylic monomers such as acids, esters or amides, or vinyl acetate or acrylonitrile especially in the presence of water.

United States Patent No. 5,254,760 discloses the use of selected nitroxide compounds in combination with an aromatic nitro compound for stabilizing vinyl aromatic monomers such as styrene. Again, there is no mention of aliphatic vinyl compounds or of the especial effectiveness of some selected nitroxide compounds in preventing the premature polymerization of such aliphatic vinyl monomers in the presence of water.

EP 697,386 generically discloses the use of selected nitroxyl compounds for preventing the premature polymerization of aromatic vinyl monomers such as styrene or aliphatic vinyl monomers such as acrylic monomers. Specifically, this reference teaches that 1-oxyl-4-acetylamino-2,2,6,6-tetramethylpiperidine alone or in combination with p-nitrosophenol or 2-methyl-4-nitrosophenol is effective in stabilizing styrene from premature polymerization. There is no mention that 1-oxyl-4-acetylamino-2,2,6,6-tetramethylpiperidine is used with an aliphatic vinyl monomer alone, and certainly no suggestion that said 1-oxyl-4-acetylamino-2,2,6,6-tetramethylpiperidine would be particularly effective with such aliphatic vinyl monomers in the presence of water.

EP 810,196 discloses the use inter alia of 1-oxyl-2,2,6,6-tetramethyl-4-acetylaminopiperidine in combination with a phosphine, such as triphenylphosphine, or a cobalt compound, such as cobalt acetate, as inhibitors to prevent the polymerization of (meth)acrylic acid or esters thereof. There is no teaching that 1-oxyl-2,2,6,6-tetramethyl-4-acetylaminopiperidine alone would be efficacious for that purpose.

Since, during the processes to produce and purify various ethylenically unsaturated monomers, water is often present during one of the process steps, there is a long felt need for the stable nitroxide inhibitor to be sufficiently water soluble or miscible to remain homogeneous in wet monomer streams and to prevent polymerization in the aqueous phase and yet for the inhibitor to be able to partition to such an extent that it can prevent polymerization in both the aqeuous phase and in the organic monomer phase for inhibition protection throughout the entire process.

The object of this invention is to provide derivatives of 1-oxyl-2,2,6,6-tetramethyl-4-hydroxypiperidine of sufficient water solubility and the concomitant ability to partition into an organic phase which will prevent the premature polymerization of ethylenically unsaturated monomers in the presence of water.

Another object of this invention to provide novel nitroxide compounds of value in stabilizing unsaturated monomers.

The instant invention is to a monomer composition stabilized against premature polymerization in the presence of water which comprises
(A) an ethylenically unsaturated monomer which is an unsaturated acid, an unsaturated ester, an unsaturated amide, an unsaturated nitrile, unsaturated ether, vinyl pyridine, diethyl vinylphosphonate or sodium styrenesulfonate, and
(B) an effective stabilizing amount of a compound of formula II 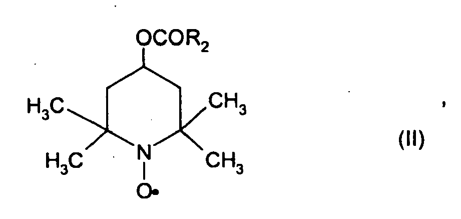
wherein
R₂ is alkyl of 3 to 5 carbon atoms interrupted by -COO- or by -CO, or R₂ is - CH₂(OCH₂CH₂)ₚOCH₃ where p is 1 to 4; or
R₂ is -NHR₃ where R₃ is alkyl of 1 to 4 carbon atoms.

The alkyl groups in the different substituents may be linear or branched.

Examples for alkyl of 1 to 6 carbon atoms are methyl ethyl propyl and its isomers, butyl and its isomers pentyl and its isomers and hexyl and its isomers.

Preferably in the compound of formula II, R₂ is 2-methoxyethoxymethyl, 2-(2-methoxyethoxy)ethoxymethyl, -CH₂COCH₃, -CH₂CH₂COOCH₃ or butylamino.

The amount of water is preferably 0.1% to 99%, more preferred 1% to 40% by weight based on the total composition.

The monomers of component (A) have at least one carbon-carbon double bond capable of undergoing free radical induced polymerization. Such monomers are well-known in commerce and comprise a wide variety of structural types. Typical examples of such monomers are the unsaturated acids such as acrylic acid, methacrylic acid and crotonic acid; unsaturated esters such as the acrylates and methacrylates exemplified by butyl acrylate, methyl methacrylate, ethyl acrylate, methyl acrylate and vinyl acetate; unsaturated amides such as acrylamide and methacrylamide: unsaturated nitriles such as acrylonitrile and methacrylonitrile; unsaturated ethers such as methyl vinyl ether; and miscellaneous vinyl monomers such as the vinyl pyridines, diethyl vinylphosphonate and sodium styrenesutfonate.

Preferably the monomer is acrylic acid, methacrylic acid, butyl acrylate, ethyl acrylate, methyl methacrylate, vinyl acetate, acrylamide or acrylonitrile; most preferably acrylic acid, vinyl acetate or acrylonitrile; most especially acrylic acid.

The effective stabilizing amount of component (B) is 1 to 10000 ppm by weight based on the weight of monomer of component (A). Preferably, the amount of component (B) is 1 to 2000 ppm by weight based on the monomer of component (A). Most preferably, the amount of component (B) is 1 to 1000 ppm by weight based on the monomer of component (A).

Preferred compounds of formula II are:
(a) 1-oxyl-2,2,6,6-tetramethylpiperidin-4-yl 2-methoxyethoxyacetate;
(b) 1-oxyl-2,2,6,6-tatramethylpiperidin-4-yl 2-(2-methoxyethoxy)ethoxyacetate;
(c) 1-oxyl-2,2,6,6-tetramethyl-piperidin-4-yl butylcarbamate;
(d) 1-oxyl-2,2,6,6-tetramethyl-piperidin-4-yl methylsuccinate.

The instant invention also pertains to a process for preventing the premature polymerization of an unsaturated monomer (A) which is an unsaturated acid, an unsaturated ester, an unsaturated amide, an unsaturated nitrile, unsaturated ether, vinyl pyridine, diethyl vinylphosphonate or sodium styrenesulfonate, in the presence of water by incorporating therein an effective stabilizing amount of a compound (8) of formula II.

The definitions in the composition as well as their preferences apply also for the process of inhibiting premature polymerization.

The polymerization inhibitor amide, ether or ester can be introduced into the monomer to be protected by any conventional method. It may be added just upstream of the point of desired application by any suitable means. In addition, this mixture may be injected separately into the distillation train along with the incoming feed of monomer or through separate entry points providing efficient distribution of the activated inhibitor mixture. Since the inhibitor is gradually depleted during operation, it is generally necessary to maintain the appropriate amount of the inhibitor ester in the distillation system by adding additional inhibitor during the course of the distillation process. Such addition may be carried out either on a continuous basis or by intermittently charging fresh inhibitor into the distillation system if the concentration of the inhibitor is to be maintained above the minimum required level.

The nitroxides of this invention are highly water compatible. As many of the processes needed to produce and purify the various ethylenically unsaturated monomers may have some water present during one of the process steps, it is important that the instant stable nitroxide inhibitor be sufficiently water soluble to prevent polymerization in the aqueous phase and yet for the inhibitor to be able to partition significantly into the organic monomer phase for inhibition protection throughout the entire process. Undesired premature polymerization must be limited or mitigated throughout the purification process to insure that the reactors, tanks and pipes used to make, store and transport the purified monomer remain free from high molecular weight polymeric material. The instant amide ether or ester inhibitors are tailored to have the desirable water compatibility properties needed to bring this about.

The amount of water present will depend on the specific monomer being stabilized. In the case of monomers of limited compatibility with water such as butyl acrylate, the water content will depend on the amount needed to saturate the ester, only a few percent. On the other hand with water miscible monomers such as acrylic acid, the amount of water possible is theoretically much higher.

A further subject of the invention is the use of a compound formula II for preventing the premature polymerization of an unsaturated monomer.

The compounds of formula II can be prepared with standard methods of organic chemistry. The intermediates are partially commercially available.

The following examples are meant to illustrate the instant invention.

### Example 1

### 1 -Oxyl-2.2,6,6-tetramethylpiperidin-4-yl-2-methoxyethoxyacetate

34.4 g of 1-oxyl-2,2,6,6-tetramethyl-4-hydroxypiperidine, 29.6 g of methyl 2-methoxyethoxyacetate and 300 ml of heptane are transferred to a 500 ml 3-necked, roundbottomed flask equipped with a mechanical stirrer, Dean-Stark trap and condenser. Trace amounts of water are removed by azeotropic distillation. 0.25 ml of tetraisopropyl orthotitanate is added to the reaction mixture. The reaction mixture is refluxed for six hours and the liberated methanol is collected in the Dean-Stark trap. The reaction mixture is allowed to cool and is then partitioned between 300 ml of ethyl acetate and 300 ml of water. The phases are separated and the organic phase is washed with water and dried over anhydrous magnesium sulfate. Evaporation of the solvent leaves the title compound as a red oil.

### Example 2

### 1-Oxyl-2,2,6,6-tetramethylpiperidin-4-yl 2-(2-methoxyethoxy)ethoxyacetate

The title compound is synthesized using the same procedure as described in Example 2 and using methyl 2-(2-methoxyethoxy)ethyoxyacetate in place of methyl 2-methoxyethoxyacetate. The title compound is isolated as a red oil after column chromatography.

### Example 3

### 1-Oxyl-2,2,6,6-tetramethyl-piperidin-4-yl-butylcarbamate

0.1 g of di-n-butyltin dilaurate is added to a solution of 1.0 g (5.8 mmol) of 1-oxyl-2,2,6,6-tetramethyl-4-hydroxypiperldine and 0.58 g (5.8 mmol) of butyl lsocyanate in 10 ml of carbon tetrachloride. After stirring for four hours at ambient temperature, the solution is concentrated and the title compound is isolated as a red oil after column chromatography.

### Example 4

### 1-Oxyl-2,2,6,6-tetramethylpiperidin-4-yl Methyl Succinate

A solution of 6.0 g (35 mmol) of 1-oxyl-2,2,6,6-tetramethyl-4-hydroxypiperidine and 11.4 g (78 mmol) dimethyl succinate in 60 mL of heptane is brought to reflux. 0.05 mL of tetraisopropyl orthotitanate is added and the reaction mixture is refluxed for 16 hours while the evolved methanol is trapped in a Dean-Stark trap. The reaction mixture is then concentrated and the title compound is isolated as a red oil after column chromatography and melts at 76°C.

In the Application Examples two different test methods are employed to determine the effectiveness of the nitroxide esters as inhibitors. The method is chosen to simulate different aspects of the purification processes.

### Method 1

Acrylic acid is distilled to remove any storage stabilizer present. Stock stabilizer solutions (1.5 mg*l*ml) are prepared in propionic acid. This stock solution is added to the distilled acrylic acid to give a test solution having 5 ppm of total stabilizer. Aliquots of this test solution are then placed into three separate reaction tubes. Each tube is purged with a gas mixture (0.65% oxygen in nitrogen) for ten minutes. The tubes are then sealed and placed in a 110 o C oil bath. The tubes are watched till the appearance of visible polymer formation is observed as a precipitate. Failure times are reported as an average of at least three tubes.

### Method 2

Test solutions are prepared as in Method 1 except that the stock stabilizer solution is prepared at 0.75 mg*l*ml giving a test solution with 2.5 ppm of total stabilizer. Aliquots (1 ml) of the test solution are placed into three separate reaction tubes. To each tube is added 0.5 ml of toluene and 0.5 ml of distilled water. Each tube is purged as described in Method 1 and then sealed. The tubes are placed in a 90°C oil bath and heated till visible polymer is observed as a precipitate. Failure times are reported as an average of at least three tubes.

### Example 5

Following the procedure of Method 1, it is seen that water miscible nitroxides and hydrophobic nitroxides each perform similarly in neat acrylic acid in the absence of water.

**Table 1**

| Stabilization of Neat Acrylic Acid | |
|---|---|
| Compound* of Example (5 ppm by weight) | Time to Onset of Polymerization (minutes) |
| none | 5 |
| A | 220 |
| Example 1 | 220 |

| | |
|---|---|
| *A is bis(1-oxyl-2,2,6,6-tetramethylpiperidin-4-yl) sebacate. | |

Each of the nitroxide compounds provide nearly the same stabilization efficace to neat acrylic acid.

### Example 6

Following the procedure of Method 2 where water is present in the acrylic acid, there is a clear difference in the superior stabilization performance of the instant water compatible nitroxides formula II compared to the hydrophobic nitroxides as seen in Table 2.

**Table 2**

| Stabilization of Aqueous Acrylic Acid | |
|---|---|
| Compound* of Example (5 ppm by weight) | Time to Onset of Polymerization (minutes) |
| none | 30 |
| A | 240 |
| B | 130 |
| Example 1 | 325 |
| Example 2 | 520 |
| Example 3 | 410 |
| Example 4 | 600 |

| | |
|---|---|
| *A is bis(1-oxyl-2,2,6,6-tetramethylpiperidin-4-yl-) sebacate. B is 1-oxyl-2,2,6,6-tetramethylpiperidin-4-yl octanoate. | |

## Claims

1. A monomer composition stabilized against premature polymerization in the presence of water which comprises
(A) an ethylenically unsaturated monomer which is an unsaturated acid, an unsaturated ester, an unsaturated amide, an unsaturated nitrile, unsaturated ether, vinyl pyridine, diethyl vinylphosphonate or sodium styrenesulfonate, and
(B) an effective stabilizing amount of a compound of formula II 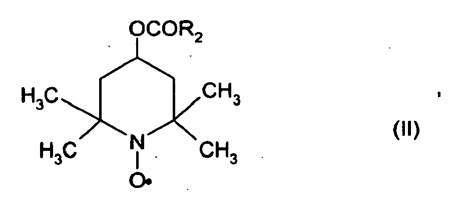
wherein
R₂ is alkyl of 3 to 5 carbon atoms interrupted by -COO- or by -CO, or R₂ is-CH₂(OCH₂CH₂)ₚOCH₃ where p is 1 to 4; or
R₂ is -NHR₃ where R₃ is alkyl of 1 to 4 carbon atoms.

2. A composition according to claim 1 where in the compound of formula II, R₂ is 2-methoxyethoxymethyl, 2-(2-methoxyethoxy)ethoxymethyl, -CH₂COCH₃, -CH₂CH₂COOCH₃ or butylamino.

3. A composition according to claim 1 wherein the amount of water is 0.1 % to 99% by weight based on the total composition.

4. A composition according to claim 1 wherein the unsaturated monomer is acrylic acid, methacrylic acid, an ester of acrylic acid or methacrylic acid, an amide of acrylic acid or methacrylic acid, vinyl acetate or acrylonitrile.

5. A composition according to claim 4 wherein the unsaturated monomer is acrylic acid, methacrylic acid, butyl acrylate, ethyl acrylate, methyl methacrylate, vinyl acetate, acrylamide or acrylonitrile.

6. A composition according to claim 1 wherein the effective stabilizing amount of component (B) is 1 to 10000 ppm by weight based on the weight of monomer of component (A).

7. A composition according to claim 1 wherein the compound of formula II is
(a) 1-oxyl-2,2,6,6-tetramethylpiperidin-4-yl 2-methoxyethoxyacetate;
(b) 1-oxyl-2,2,6,6-tetramethylpiperidin-4-yl 2-(2-methoxyethoxy)ethoxyacetate; or
(c) 1-oxyl-2,2,6,6-tetramethyl-piperidin-4-yl butylcarbamate.
(d) 1-oxyl-2,2,6,6-tetramethyl-piperidin-4-yl methylsuccinate

8. A process for preventing the premature polymerization of an unsaturated monomer (A) which is an unsaturated acid, an unsaturated ester, an unsaturated amide, an unsaturated nitrile, unsaturated ether, vinyl pyridine, diethyl vinylphosphonate or sodium styrenesulfonate, in the presence of water by incorporating therein an effective stabilizing amount of a compound (B) of formula II according to claim 1.

9. Use of a compound of formula II according to claim 1 for preventing the premature polymerization of an unsaturated monomer.

## Patentansprüche

1. Monomer-Zusammensetzung, stabilisiert gegen vorzeitige Polymerisation in Gegenwart von Wasser, die umfasst
(A) ein ethylenisch ungesättigtes Monomer, das eine ungesättigte Säure, ein ungesättigter Ester, ein ungesättigtes Amid, ein ungesättigtes Nitril, ungesättigter Ether, Vinylpyridin, Diethyl-vinylphosphonat oder Natrium-styrolsulfonat ist, und
(B) eine wirksam stabilisierende Menge von einer Verbindung der Formel II 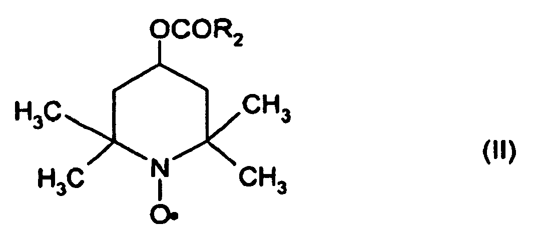
worin
R₂ Alkyl mit 3 bis 5 Kohlenstoffatomen, unterbrochen durch -COO- oder durch -CO, darstellt, oder R₂ -CH₂(OCH₂CH₂)ₚOCH₃ darstellt, worin p 1 bis 4 ist; oder
R₂ -NHR₃ darstellt, worin R₃ Alkyl mit 1 bis 4 Kohlenstoffatomen darstellt.

2. Zusammensetzung nach Anspruch 1, worin in der Verbindung der Formel II, R₂ 2-Methoxyethoxymethyl, 2-(2-Methoxyethoxy)-ethoxymethyl, -CH₂COCH₃, -CH₂CH₂COOCH₃ oder Butylamino darstellt.

3. Zusammensetzung nach Anspruch 1, worin die Wassermenge 0,1 % bis 99 Gew.-%, bezogen auf die gesamte Zusammensetzung, ist.

4. Zusammensetzung nach Anspruch 1, worin das ungesättigte Monomer Acrylsäure, Methacrylsäure, ein Ester von Acrylsäure oder Methacrylsäure, ein Amid von Acrylsäure oder Methacrylsäure, Vinylacetat oder Acrylnitril ist.

5. Zusammensetzung nach Anspruch 4, worin das ungesättigte Monomer Acrylsäure, Methacrylsäure, Acrylsäurebutylester, Acrylsäureethylester, Methacrylsäuremethylester, Vinylacetat, Acrylamid oder Acrylnitril ist.

6. Zusammensetzung nach Anspruch 1, worin die wirksame stabilisierende Menge von Komponente (B) 1 bis 10 000 ppm auf das Gewicht, bezogen auf das Gewicht von Monomer von Komponente (A), ist.

7. Zusammensetzung nach Anspruch 1, worin die Verbindung der Formel II bedeutet
(a) 1-Oxyl-2,2,6,6-tetramethyl-piperidin-4-yl-2-methoxyethoxyacetat;
(b) 1-Oxyl-2,2,6,6-tetramethyl-piperidin-4-yl-2-(2-methoxyethoxy)-ethoxyacetat;
(c) 1-Oxyl-2,2,6, 6-tetramethyl-piperidin-4-yl-butyl-carbamat; oder
(d) 1-Oxyl-2,2,6,6-tetramethyl-piperidin-4-yl-methyl-succinat.

8. Verfahren zur Verhinderung der vorzeitigen Polymerisation von einem ungesättigten Monomer (A), das eine ungesättigte Säure, ein ungesättigter Ester, ein ungesättigtes Amid, ein ungesättigtes Nitril, ungesättigter Ether, Vinylpyridin, Diethyl-vinylphosphonat oder Natrium-styrolsulfonat ist, in Gegenwart von Wasser durch Einarbeiten darin einer wirksam stabilisierenden Menge von einer Verbindung (B) der Formel II nach Anspruch 1.

9. Verwendung einer Verbindung der Formel II nach Anspruch 1 zur Verhinderung der vorzeitigen Polymerisation von einem ungesättigten Monomer.

## Revendications

1. Composition monomérique stabilisée à l'encontre de la polymérisation prématurée en présence d'eau qui comprend
a. un monomère éthyléniquement insaturé qui est un acide insaturé, un ester insaturé, un amide insaturé, un nitrile insaturé, un éther insaturé, une vinyl pyridine, un vinylphosphonate de diéthyl ou un styrènesulfonate de sodium, et
b. une quantité stabilisante efficace d'un composé de formule (II) 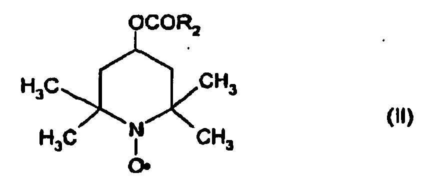
dans laquelle,
R₂ est alkyle possédant de 3 à 5 atomes de carbone interrompu par - COO- ou par -CO, ou R₂ est -CH₂(OCH₂CH₂)ₚOCH₃ dans laquelle, p est de 1 à 4 ; ou
R₂ est -NHR₃ dans laquelle, R₃ est alkyle possédant de 1 à 4 atomes de carbone.

2. Composition selon la revendication 1, **caractérisée en ce que** dans le composé de formula II, R₂ est 2-méthoxy-éthoxyméthyle, 2-(2-méthoxyéthoxy)éthoxyméthyle, -CH₂COCH₃, -CH₂CH₂COOCH₃ ou butylamino.

3. Composition selon la revendication 1, **caractérisée en ce que** la quantité d'eau va de 0, 1 % à 99% en poids sur base de la composition totale.

4. Composition selon la revendication 1, **caractérisée en ce que** le monomère insaturé est de l'acide acrylique, de l'acide méthacrylique, un ester d'acide acrylique ou d'acide méthacrylique, un amide d'acide acrylique ou d'acide méthacrylique, un acétate de vinyle ou un acrylonitrile.

5. Composition selon la revendication 4, **caractérisée en ce que** le monomère insaturé est de l'acide acrylique, de l'acide méthacrylique, du butyl acrylate, de l'éthyl acrylate, du méthyl méthacrylate, du vinyl acétate, de l'acrylamide ou un acrylonitrile.

6. Composition selon la revendication 1, **caractérisée en ce que** la quantité stabilisante efficace du composant (B) est comprise dans la gamme allant de 1 à 10 000 ppm en poids sur base du poids du monomère du composant (A).

7. Composition selon la revendication 1, **caractérisée en ce que** le composé de formule II est
a. 2-méthoxyéthoxyacétate de 1-oxyl-2,2,6,6-tétraméthylpipéridin-4-yle ;
b. 2-(2-méthoxyéthoxy)éthoxyacétate de 1-oxyl-2,2,6,6-tétraméthyl-pipéridin-4-yle ;
c. butylcarbamate de 1-oxyl-2,2,6,6-tétraméthyl-pipéridin-4-yle ;
d. méthyl succinate de 1-oxyl-2,2,6,6-tétraméthyl-pipéridin-4-yle.

8. Procédé de prévention à l'encontre de la polymérisation prématurée d'un monomère insaturé (A) qui est un acide insaturé, un ester insaturé, un amide insaturé, un nitrile insaturé, un éther insaturé, une vinyl pyridine, un vinylphosphonate de diéthyle, ou un styrènesulfonate de sodium en présence d'eau qui consiste à y incorporer une quantité stabilisante efficace du composant (B) de formule II selon la revendication 1.

9. Utilisation d'un composé de formule II selon la revendication 1 pour la prévention à l'encontre de la polymérisation prématurée d'un monomère insaturé.
